# EUROPEAN PATENT APPLICATION

(11) **EP 4 817 647 A1**
(43) Date of publication of application: **07.10.2026**
(21) Application number: 24900573.7
(22) Date of filing: 02.12.2024
(51) Int. Cl.: A61L 29/06, A61L 29/04, A61L 29/12, A61L 29/14, A61L 31/04, A61L 31/06, A61L 31/12, A61L 31/14, A61M 25/00

(54) **MEDICAL INSTRUMENT AND MEDICAL INSTRUMENT MANUFACTURING METHOD**

(30) Priority: 06.12.2023 JP 2023205991
(71) Applicant: Terumo Kabushiki Kaisha, Tokyo 151-0072 (JP)
(72) Inventor: WATANABE, Nozomu, Fujinomiya-shi, Shizuoka 418-0015 (JP)
(74) Representative: Casalonga
(86) International application number: PCT/JP2024/042497
(87) International publication number: WO 2025/121279

(57) **Abstract**

Provided is a medical device having a surface lubricating layer that exhibits an excellent lubrication retaining property even in a drying process after wetting.

A medical device according to the present invention includes a base layer, and a surface lubricating layer supported on at least a part of the base layer, in which the surface lubricating layer contains a block copolymer having a structural unit (A) derived from a reactive monomer having an epoxy group and a structural unit (B) derived from a hydrophilic monomer, and a silicone compound, and when 1.0 µL of purified water is added dropwise to the surface lubricating layer, and a water contact angle after 5 seconds from contact between a water droplet of the purified water and the surface lubricating layer is θ1 and a water contact angle after 5 minutes from the contact is θ2, the θ1 is 104° or more, and the θ2 is 102° or less.

## Description

### Technical Field

The present invention relates to a medical device and a method of manufacturing a medical device.

### Background Art

Medical devices inserted into a living body such as a catheter, a guide wire, and an indwelling needle are required to exhibit an excellent lubricating property in order to reduce damage to tissues such as blood vessels and improve operability of an operator. Thus, a method of coating a base layer surface with a hydrophilic polymer having the lubricating property has been developed and put to practical use. In such a medical device, elution and peeling of a hydrophilic polymer from the base layer surface are problems in terms of safety and maintenance of operability. Therefore, coating with a hydrophilic polymer is required not only to have the excellent lubricating property but also to have durability against loads such as wear and abrasion.

From such a viewpoint, JP H8-33704 A (corresponding to US 5670558) discloses a medical device in which a water-soluble or water-swellable polymer is dissolved in a solvent in which a base material of the medical device swells to prepare a polymer solution, the base material of the medical device is immersed in the polymer solution to swell, and the polymer is crosslinked or polymerized on a base material surface to form a surface lubricating layer on the base material surface. According to the technique disclosed in JP H8-33704 A, a surface lubricating layer exhibiting a relatively good lubricating property can be immobilized to a base material.

### Summary of Invention

JP H8-33704 A discloses that it is preferable to use, as a water-soluble or water-swellable polymer, a block copolymer composed of a hydrophilic moiety exhibiting lubricating property and a moiety having an epoxy group. When such a block copolymer is used, the epoxy group of the block copolymer can be crosslinked through a heating operation, and a surface lubricating layer that is relatively difficult to peel off can be formed.

However, the present inventors have found that the surface lubricating layer formed by using the block copolymer exhibits excellent surface lubricating property in a state of being wetted with water (for example, physiological saline), but has a problem that when the surface gradually dries with time, the surface becomes sticky. As described above, when the adhesiveness is developed with the drying of the surface, the operability of the medical device greatly deteriorates. For example, in a surface of a medical device such as a catheter or a guide wire, in particular, in a region (mainly on a base portion side) that enters or exits in another device such as an introducer sheath or a catheter, the surface of the medical device may be dried over time during a procedure, and may be stuck to another device or the like by being sticky as described above. Therefore, in order to avoid this, it is necessary to perform an operation of maintaining a wet state of a medical device surface by using, for example, a method of spraying water (for example, physiological saline) on the medical device surface (surface lubricating layer) or a method of wetting the medical device surface with a wet cloth.

However, since such an operation becomes a work load during a procedure, a medical device capable of maintaining high lubricating property without exhibiting adhesiveness even in a drying process after a medical device surface (surface lubricating layer) is in a wet state is required.

The present invention has been made in view of the above circumstances, and an object of the present invention is to provide a medical device having a surface lubricating layer that exhibits excellent lubrication retaining property even in a drying process after wetting. Another object of the present invention is to provide a method of manufacturing the medical device.

The present inventor has intensively studied to solve the above problems. As a result, the present inventor has found that the above object can be achieved by forming a surface lubricating layer containing a hydrophilic block copolymer having an epoxy group and a silicone compound and setting a water contact angle of a surface of the surface lubricating layer within a specific range, and has completed the present invention.

The above object can be achieved by the present invention having the following configuration, and the present invention includes the following aspects and forms.

One aspect of the present invention provides the following medical device.
1. A medical device including a base layer; and a surface lubricating layer supported on at least a part of the base layer, in which
   the surface lubricating layer contains a block copolymer having a structural unit (A) derived from a reactive monomer having an epoxy group and a structural unit (B) derived from a hydrophilic monomer, and a silicone compound, and
   when 1.0 µL of purified water is added dropwise to the surface lubricating layer, and a water contact angle after 5 seconds from contact between a water droplet of the purified water and the surface lubricating layer is θ1 and a water contact angle after 5 minutes from the contact is θ2,
   θ1 is 104° or more, and θ2 is 102° or less.
2. In the medical device according to the above 1, when a water contact angle after 1 minute from the contact between the water droplet of the purified water and the surface lubricating layer is θ3,
   θ3 preferably exceeds 102°.
3. In the medical device according to the above 1 or 2, the surface lubricating layer preferably contains the silicone compound and the block copolymer at a mass ratio of 0.1 to 1 : 1.
4. In the medical device according to any one of above 1 to 3, the silicone compound preferably contains reactive silicone.
5. In the medical device according to the above 4, the reactive silicone preferably has at least one selected from the group consisting of a hydroxyl group (-OH), a carboxyl group, an amino group (-NH₂), an epoxy group, an ethylenically unsaturated group, and an alkoxysilyl group.
6. In the medical device according to any one of above 1 to 5, the reactive monomer having an epoxy group preferably contains at least one selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether.
7. In the medical device according to any one of above 1 to 6, the hydrophilic monomer preferably contains at least one selected from the group consisting of N,N-dimethylacrylamide, acrylamide, 2-hydroxyethyl methacrylate, and N-vinylpyrrolidone.

Another aspect of the present invention provides the following method of manufacturing a medical device.

8. A method of manufacturing a medical device including a base layer and a surface lubricating layer supported on at least a part of the base layer, the method comprising:
preparing a coating liquid containing a block copolymer having a structural unit (A) derived from a reactive monomer having an epoxy group and a structural unit (B) derived from a hydrophilic monomer, a silicone compound, and a solvent; and
applying the coating liquid onto the base layer.

9. In the method of manufacturing a medical device according to the above 8, the coating liquid preferably contains the silicone compound in an amount of 0.5 to 5% by mass.

10. In the method of manufacturing a medical device according to the above 8 or 9, the silicone compound preferably contains reactive silicone.

11. In the method of manufacturing a medical device according to the above 10, the reactive silicone preferably has at least one selected from the group consisting of a hydroxyl group (-OH), a carboxyl group, an amino group (-NH₂), an epoxy group, an ethylenically unsaturated group, and an alkoxysilyl group.

12. In the method of manufacturing a medical device according to any one of above 8 to 11, the solvent preferably contains at least one selected from the group consisting of methanol, ethanol, isopropyl alcohol, butanol, ethylene glycol, acetone, methyl ethyl ketone, cyclohexanone, ethyl acetate, butyl acetate, tetrahydrofuran (THF), butyl ether, dioxane, hexane, heptane, benzene, toluene, dimethyl sulfoxide (DMSO), and N,N-dimethylformamide (DMF).

13. In the method of manufacturing a medical device according to any one of above 8 to 12, the reactive monomer having an epoxy group preferably contains at least one selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether.

14. In the method of manufacturing a medical device according to any one of the above 8 to 13, the hydrophilic monomer preferably contains at least one selected from the group consisting of N,N-dimethylacrylamide, acrylamide, 2-hydroxyethyl methacrylate, and N-vinylpyrrolidone.

### Brief Description of Drawings

[Fig. 1] Fig. 1 is a partial cross-sectional view schematically illustrating a laminated configuration of a surface of a representative embodiment of a medical device according to the present invention.
[Fig. 2] Fig. 2 is a partial cross-sectional view schematically illustrating a different configuration example of the laminated configuration of the surface as an application example of the embodiment of Fig. 1.
[Fig. 3] Fig. 3 is a graph illustrating a result of a pinch test (Wet-Dry test) in a drying process after wetting for each sample in Examples and Comparative Examples.
[Fig. 4] Fig. 4 is a graph illustrating a result of a pinch test (Dry test) in a dry state (in air) for each sample in Examples and Comparative Examples.
[Fig. 5] Fig. 5 is a graph illustrating a result of a pinch test (Wet test) in a wet state (in water) for each sample in Examples and Comparative Examples.
[Fig. 6] Fig. 6 is a schematic diagram illustrating a test device for performing a sliding test of a medical device according to the present embodiment, and is a diagram illustrating a state in which a pair of contact members sandwich the medical device.
[Fig. 7] Fig. 7 is a schematic diagram illustrating a test device for performing a sliding test of a medical device according to the present embodiment, and is a diagram illustrating a state in which the medical device is separated from a pair of contact members.

### Description of Embodiments

A medical device according to an aspect of the present invention is a medical device including a base layer and a surface lubricating layer supported on at least a part of the base layer, in which the surface lubricating layer includes a block copolymer having a structural unit (A) derived from a reactive monomer having an epoxy group and a structural unit (B) derived from a hydrophilic monomer, and a silicone compound. In the medical device according to one aspect of the present invention, when 1.0 µL of purified water is added dropwise to the surface lubricating layer, a water contact angle after 5 seconds from contact between a water droplet of the purified water and the surface lubricating layer is θ1, and a water contact angle after 5 minutes from the contact is θ2, θ1 is 104° or more, and θ2 is 102° or less. Hereinafter, the medical device having the above configuration will also be referred to as "medical device according to the present invention" or "medical device".

In the present specification, the structural unit (A) derived from a reactive monomer having an epoxy group is also simply referred to as a "structural unit (A) according to the present invention" or "structural unit (A)". In the present specification, the structural unit (B) derived from a hydrophilic monomer is also simply referred to as a "structural unit (B) according to the present invention" or "structural unit (B)". In the present specification, the block copolymer having the structural units (A) and (B) is also simply referred to as "block copolymer according to the present invention "," hydrophilic block copolymer ", or "block copolymer".

In the present specification, when a structural unit is defined as being "derived" from a certain monomer, this indicates that the structural unit is a structural unit generated by cleavage of one bond of a polymerizable unsaturated double bond of the corresponding monomer.

In the present specification, the term "(meth)acryl" refers to both acryl and methacryl. Therefore, for example, the term "(meth)acrylic acid" refers to both acrylic acid and methacrylic acid. Similarly, the term "(meth)acryloyl" refers to both acryloyl and methacryloyl. Therefore, for example, the term "(meth)acryloyl group" refers to both acryloyl group and methacryloyl group. Further similarly, the term "(meth)acrylate" refers to both acrylate and methacrylate. For example, the term "alkoxyalkyl (meth)acrylate" refers to both alkoxyalkyl acrylate and alkoxyalkyl methacrylate.

In the present specification, a range from "X to Y" includes X and Y, and indicates "X or more and Y or less". "X and/or Y" indicates at least one of X and Y, and includes X alone, Y alone, and a combination of X and Y.

Unless otherwise specified, operations and measurements of physical properties and the like are performed under the conditions of room temperature (20 to 25°C)/relative humidity 40 to 50%RH.

A medical device according to the present invention has a surface lubricating layer containing a block copolymer having a structural unit (A) derived from a reactive monomer having an epoxy group and a structural unit (B) derived from a hydrophilic monomer, and a silicone compound, and a water contact angle of a surface of the surface lubricating layer is within a specific range. Specifically, in the medical device according to the present invention, 1.0 µL of purified water is added dropwise to the surface lubricating layer on the surface of the surface lubricating layer, a water contact angle (θ1) after 5 seconds from contact between a water droplet of the dropped purified water and the surface lubricating layer is 104° or more, and a water contact angle (θ2) after 5 minutes from contact between the water droplet and the surface lubricating layer is 102° or less.

The present inventor has presumed that the occurrence of adhesiveness (stickiness) in the drying process after wetting is caused by a hydrophilic block copolymer that imparts good slidability to the surface lubricating layer. More specifically, the present inventor thought that when the hydrophilic block copolymer is in a wet state, the surface lubricating layer can exhibit good slidability, but when the hydrophilic block copolymer (the surface lubricating layer containing the hydrophilic block copolymer) gradually dries, the surface lubricating layer cannot exhibit slidability as in a wet state, and thus the adhesiveness (stickiness) may be generated.

On the other hand, the medical device according to the present invention further contains a silicone compound in the surface lubricating layer containing such a hydrophilic block copolymer. Here, the block copolymer contained in the surface lubricating layer functions as a hydrophilic component, while the silicone compound contained in the surface lubricating layer functions as a water-repellent component (hydrophobic component). Since the surface lubricating layer of the medical device according to the present invention contains a silicone compound, and the surface has the water contact angle θ1 (the water contact angle after 5 seconds from when the surface lubricating layer comes into contact with a water droplet) of a specific value or more, it can be said that the surface lubricating layer has moderate water repellency (hydrophobicity) immediately after coming into contact with the water droplet.

Such moderate water repellency is presumed to be due to the silicone compound being present on the surface of the surface lubricating layer, and the proportion of the hydrophilic block copolymer on the surface being moderately small.

Therefore, on the surface of the surface lubricating layer of the medical device according to the present invention, since the proportion of the hydrophilic block copolymer is appropriately small as described above, the influence (that is, the occurrence of adhesiveness) due to drying of the hydrophilic block copolymer can be reduced. Therefore, according to the present invention, it is possible to provide a medical device having a surface lubricating layer that exhibits excellent lubrication retaining property even in a drying process after wetting.

Furthermore, in the surface lubricating layer of the medical device according to the present invention, the water contact angle θ2 (the water contact angle after 5 minutes from when the surface lubricating layer comes into contact with a water droplet) is a specific value or less. Such physical properties are considered to be due to after the surface lubricating layer comes into contact with water (after becoming wet), hydrophilicity by the hydrophilic block copolymer contained in the surface lubricating layer is exhibited with the lapse of time. Therefore, the surface lubricating layer can maintain good hydrophilicity while containing a silicone compound, and as a result, can exhibit high lubricating property (surface lubricating property).

As described above, the medical device according to the present invention can obtain a desired effect of maintaining excellent lubricating property (surface lubricating property) while avoiding the problem of occurrence of adhesiveness (stickiness) in a drying process after wetting. Note that the above mechanism is an inference and does not limit the technical scope of the present invention.

Hereinafter, embodiments of the present invention will be described. Note that the present invention is not limited only to the following embodiments, and various modifications can be made within the scope of claims. In addition, the embodiments described in the present specification can be optionally combined with each other to form another embodiment. The dimensional ratios in the drawings are exaggerated for convenience of description and may be different from actual ratios. In a case in which the embodiments of the present invention are described with reference to the drawings, the same elements are denoted by the same reference numerals in the description of the drawings, and redundant description will be omitted.

Throughout the present specification, expression of a singular should be understood to include also a concept of a plural thereof unless otherwise stated. Thus, an article of a singular (for example, "a", "an", and "the" in English) should be understood to include also a concept of a plural thereof unless otherwise stated. Terms used in the present specification should be understood to be used in a sense commonly used in the art unless otherwise stated. Thus, unless defined otherwise, all technical and scientific terms used herein have the same meaning as commonly understood by those skilled in the art to which this invention belongs. In the case of contradiction, priority is given to the present specification (including definitions).

### [Medical device]

A medical device according to the present invention includes a base layer and a surface lubricating layer supported on at least a part of the base layer. The surface lubricating layer contains a block copolymer having a structural unit (A) derived from a reactive monomer having an epoxy group and a structural unit (B) derived from a hydrophilic monomer, and a silicone compound. Further, the surface lubricating layer has the following characteristics.

When 1.0 µL of purified water is added dropwise to the surface lubricating layer, a water contact angle after 5 seconds from contact between a water droplet of the purified water and the surface lubricating layer is θ1, and a water contact angle after 5 minutes from the contact is θ2, θ1 is 104° or more, and θ2 is 102° or less.

Hereinafter, a preferred embodiment of a medical device according to the present invention will be described with reference to the accompanying drawings.

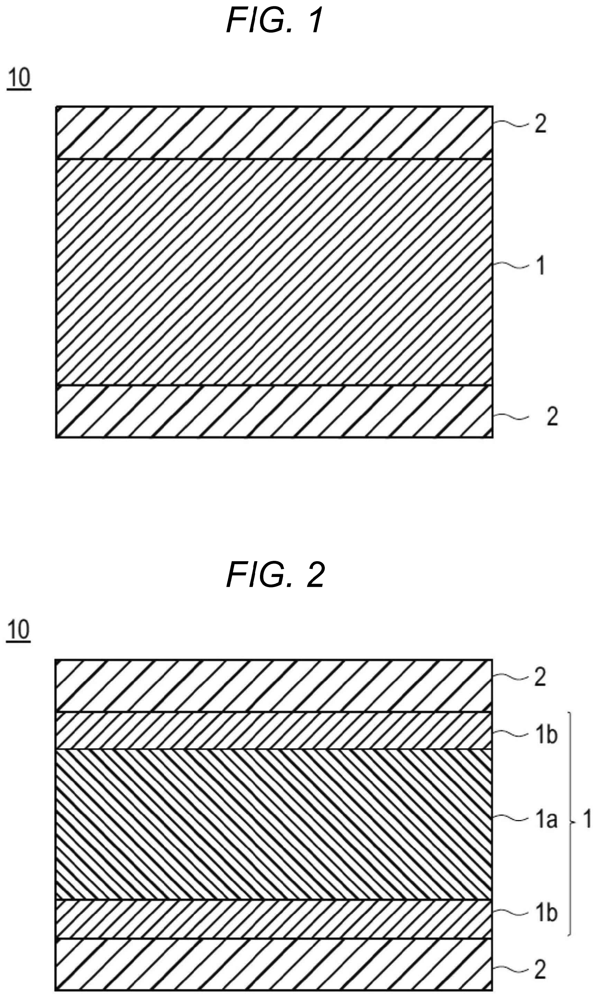

Fig. 1 is a partial cross-sectional view schematically illustrating a laminated structure of a surface of a representative embodiment of a medical device according to the present invention. Fig. 2 is a partial cross-sectional view schematically illustrating a different configuration example of the laminated structure of the surface as an application example of the embodiment illustrated in Fig. 1. Note that reference numerals in Fig. 1 and Fig. 2 indicate the following. The reference numeral 1 denotes a base layer; the reference numeral 1a denotes a base layer core portion; the reference numeral 1b denotes a base material surface layer; the reference numeral 2 denotes a surface lubricating layer; and the reference numeral 10 denotes a medical device according to the present invention.

As illustrated in Fig. 1 and Fig. 2, the medical device 10 of the present embodiment includes the base layer 1 and the surface lubricating layer 2 provided on at least a part of the base layer 1 and including a block copolymer (in the drawing, an example in which the film is provided on the entire surface (whole surface) of the base layer 1 in the drawing is illustrated). In Fig. 1 and Fig. 2, the surface lubricating layer 2 is formed on both surfaces of the base layer 1, but the present invention is not limited to the above embodiment, and may be any form such as a form in which it is formed on one surface of the base layer 1; and a form in which it is formed on a part of one surface or both surfaces of the base layer 1.

Hereinafter, the medical device will be described in detail for each component.

### <Base layer (Base Material)>

The base layer used in the present embodiment may be composed of any material, and the material is not particularly limited. Specific examples of the material constituting the base layer 1 include a metal material, a polymer material, and ceramics.

Among the materials constituting the base layer 1, a metal material is not particularly limited, and metal materials generally used for medical devices such as catheters, guide wires, and indwelling needles are used. Specific examples thereof include various stainless steels such as SUS304, SUS314, SUS316, SUS316L, SUS420J2, and SUS630, gold, platinum, silver, copper, nickel, cobalt, titanium, iron, aluminum, tin, or various alloys such as a nickel-titanium alloy, a nickel-cobalt alloy, a cobalt-chromium alloy, and a zinc-tungsten alloy. These may be used singly or in combination of two or more types thereof. As the metal material, an optimal metal material may be appropriately selected as a base layer of a catheter, a guide wire, an indwelling needle, or the like to be used.

In addition, among the materials constituting the base layer 1, a polymer material (a resin material or an elastomer material) is not particularly limited, and a polymer material generally used for medical devices such as a catheter, an introducer, a guide wire, and an indwelling needle is used. Specific examples thereof include a polyamide resin, polyolefin resins such as a polyethylene resin and a polypropylene resin, a modified polyolefin resin, a cyclic polyolefin resin, an epoxy resin, a polyurethane resin, a diallyl phthalate resin (allyl resin), a polycarbonate resin, a fluororesin, an amino resin (a urea resin, a melamine resin, a benzoguanamine resin), a polyethylene terephthalate resin and polyester resins such as a polybutylene terephthalate resin, a styrene resin, an acrylic resin, a polyacetal resin, a vinyl acetate resin, a phenol resin, a vinyl chloride resin, a silicone resin (silicon resin), a polyether resin and a polyimide resin.

Thermoplastic elastomers such as a polyurethane elastomer, a polyester elastomer, and a polyamide elastomer (nylon elastomer) can also be used as the material of the base layer.

These polymer materials may be used singly, or may be used as a mixture of two or more thereof or as a copolymer of two or more monomers constituting any of the resins or elastomers. As the polymer material, a polymer material optimal for a base layer of a catheter, a guide wire, an indwelling needle, or the like to be used may be selected as appropriate.

A shape of the base layer 1 is not particularly limited, and a sheet shape, a linear shape (wire), a rod shape, a tubular shape, or the like is selected as appropriate according to a use mode.

Here, in the base layer 1, the entire base layer 1 may be made of any of the above materials. The base layer 1 may be a multilayer structure formed by laminating different materials in multiple layers, a structure in which members formed of different materials are connected for each part of a medical device, or the like. Alternatively, as illustrated in Fig. 2, the base layer core portion 1a may have a structure in which the base material surface layer 1b is formed by covering the surface of the base layer core portion 1a made of any of the above materials with any other of the above materials by an appropriate method. Examples of the latter case include: the layer in which the surface of the base layer core portion 1a made of a resin material or the like is covered with a metal material by an appropriate method (conventionally known methods such as plating, metal vapor deposition, and sputtering) to form the base material surface layer 1b; and the layer in which the surface of a base layer core portion 1a made of a hard reinforcing material such as a metal material or a ceramic material is covered with a polymer material that is softer than the reinforcing material such as a metal material by an appropriate method (conventionally known methods such as immersion (dipping), spraying, and coating/printing), or the reinforcing material constituting the base layer core portion 1a and the polymer material are combined to form the base material surface layer 1b. Further, the base layer core portion 1a may have a multilayer structure formed by laminating different materials in multiple layers, a structure in which members made of different materials are connected for each part of the medical device, or the like. Another middle layer (not illustrated) may be further formed between the base layer core portion 1a and the base material surface layer 1b. The base material surface layer 1b may also have a multilayer structure formed by laminating different materials in multiple layers, a structure in which members made of different materials are connected for each part of the medical device, or the like.

### <Surface lubricating layer (cover layer)>

The surface lubricating layer is supported on at least a part of the base layer 1. Here, the reason why the surface lubricating layer 2 is supported on at least a part of the surface of the base layer 1 is that, in a medical device such as a catheter, a guide wire, or an indwelling needle to be used, it is not always necessary that all surfaces (the entire surface) of these medical devices have the lubricating property in a wet state, and it is sufficient that the surface lubricating layer is supported only on a surface portion (which may be a part or all) where the surface is required to have the lubricating property in a wet state. Therefore, as described above, the surface lubricating layer includes: a form in which the surface lubricating layer is formed to cover the entire both surfaces of the base layer as illustrated in Fig. 1 and Fig. 2; a form in which the surface lubricating layer is formed to cover only entire one surface of the base layer; a form in which the surface lubricating layer is formed to cover a part of both surfaces of the base layer in the same or different form; a form in which the surface lubricating layer is formed to cover a part of one surface of the base layer, and the like.

### (Water contact angles θ1, θ2 and θ3)

In the surface lubricating layer, when 1.0 µL of purified water is added dropwise to the surface lubricating layer, a water contact angle (θ1) after 5 seconds (a time point at which 5 seconds elapsed) from contact between the dropped water droplet and the surface lubricating layer is 104° or more. In addition, in the surface lubricating layer, when 1.0 µL of purified water is added dropwise to the surface lubricating layer in the same manner as described above, a water contact angle (θ2) after 5 minutes (a time point at which 5 minutes elapsed) from contact between the dropped water droplet and the surface lubricating layer is 102° or less. Furthermore, in the surface lubricating layer, when 1.0 µL of purified water is added dropwise to the surface lubricating layer in the same manner as described above, a water contact angle (θ3) after 1 minute (a time point at which 1 minute elapsed) from contact between the dropped water droplet and the surface lubricating layer is preferably more than 102°. In the present specification, the water contact angles θ1 and θ2 and the water contact angle θ3 that will be described later are measured under the conditions of room temperature (25°C) and a relative humidity of 50%RH, and a specific measurement method is the method described in Examples. Further, in the present specification, when 1.0 µL of purified water is added dropwise to the surface lubricating layer, the water contact angle after 5 seconds (a time point at which 5 seconds elapsed) from contact between the dropped water droplet and the surface lubricating layer, the water contact angle after 5 minutes (a time point at which 5 minutes elapsed) from the contact, and the water contact angle after 1 minute (a time point at which 1 minute elapsed) from the contact are also simply referred to as a "water contact angle θ1", a "water contact angle θ2", and a "water contact angle θ3", respectively.

From the viewpoint of effectively suppressing adhesiveness (stickiness) in a drying process after wetting, the water contact angle θ1 is preferably 105° or more, more preferably 110° or more, still more preferably 115° or more, and particularly preferably 120° or more. The upper limit value of the water contact angle θ1 is not particularly limited, but is preferably 140° or less, and more preferably 130° or less from the viewpoint of favorably maintaining slidability (surface lubricating property) in a wet state.

Therefore, in an embodiment of the present invention, the water contact angle θ1 is preferably 104 to 140°, more preferably 105 to 140°, still more preferably 110 to 140°, particularly preferably 115 to 130°, and most preferably 120 to 130°.

In addition, the water contact angle θ2 is preferably 101° or less, and more preferably 100° or less from the viewpoint of obtaining excellent slidability (surface lubricating property) in a wet state. The lower limit value of the water contact angle θ2 is not particularly limited, but is preferably 78° or more, more preferably 80° or more, still more preferably 85° or more, and particularly preferably 90° or more from the viewpoint of effectively suppressing adhesiveness (stickiness) in a drying process after wetting.

Therefore, in an embodiment of the present invention, the water contact angle θ2 is preferably 78 to 102°, more preferably 80 to 102°, still more preferably 85 to 102°, still more preferably 85 to 101°, and particularly preferably 90 to 100°. Furthermore, when the water contact angle θ2 is within any of these ranges, the water contact angle θ1 is preferably within any of the above preferred ranges.

Furthermore, from the viewpoint of effectively suppressing adhesiveness (stickiness) in a drying process after wetting, the water contact angle θ3 is more preferably 103° or more, still more preferably 105° or more, and particularly preferably 110° or more. The upper limit value of the water contact angle θ3 is not particularly limited, but is preferably 130° or less, more preferably 125° or less, and particularly preferably 120° or less from the viewpoint of favorably maintaining the slidability (surface lubricating property) in a wet state.

Therefore, in an embodiment of the present invention, the water contact angle θ3 is preferably 102 to 130°, more preferably more than 102° and 130° or less, still more preferably 103 to 130°, still more preferably 105 to 125°, and particularly preferably 110 to 120°.

Further, in another embodiment, in the surface lubricating layer, the water contact angle θ2 may be 78 to 102° and the water contact angle θ3 may be 102 to 130°. In another preferred embodiment, in the surface lubricating layer, the water contact angle θ2 may be 78 to 102° and the water contact angle θ3 may be more than 102°C and 130° or less. In another preferred embodiment, in the surface lubricating layer, the water contact angle θ2 may be 80 to 102° and the water contact angle θ3 may be 103 to 130°. In another preferred embodiment, in the surface lubricating layer, the water contact angle θ2 may be 85 to 101° and the water contact angle θ3 may be 105 to 125°. In another preferred embodiment, in the surface lubricating layer, the water contact angle θ2 may be 90 to 100° and the water contact angle θ3 may be 110 to 120°.

The water contact angles θ1, θ2, and θ3 of the surface lubricating layer can be appropriately controlled by a content ratio (mass ratio) of the block copolymer and the silicone compound contained in the surface lubricating layer, and heating and drying conditions after a coating liquid containing these components is applied onto the base layer. Details of the content ratio (mass ratio) of each component constituting the surface lubricating layer, the heating and drying conditions, and the like will be described later.

### (Block copolymer)

In the present invention, the block copolymer constitutes a surface lubricating layer supported on at least a part of the base layer. That is, in the medical device according to the present invention, the surface lubricating layer contains a block copolymer. The term "supporting" means a state in which the surface lubricating layer is fixed in a state of not being easily released from the surface of the base layer, and includes not only a form in which the entire surface of the base layer is completely covered with the surface lubricating layer, but also a form in which only a part of the surface of the base layer is covered with the surface lubricating layer, that is, a form in which the surface lubricating layer is attached to only a part of the surface of the base layer.

The block copolymer according to the present invention has the structural unit (A) derived from a reactive monomer having an epoxy group and the structural unit (B) derived from a hydrophilic monomer.

The reactive monomer having an epoxy group constituting the block copolymer has an epoxy group as a reactive group. The epoxy group is ring-opened to promote crosslinking (bonding) between the block copolymers, and the film strength of the surface lubricating layer is increased by introducing the structural unit (A) derived from such a reactive monomer into the block copolymer. In a case where the base layer is a resin material, crosslinking (bonding) between the block copolymer and the base layer may also occur due to the ring-opened epoxy group.

The reactive monomer constituting the block copolymer is not particularly limited as long as it has an epoxy group, and a known compound can be used. Among them, the reactive monomer having an epoxy group preferably contains at least one selected from the group consisting of glycidyl acrylate, glycidyl methacrylate (GMA), 3,4-epoxycyclohexyl methyl acrylate, 3,4-epoxycyclohexyl methyl methacrylate, β-methyl glycidyl methacrylate, and allyl glycidyl ether because crosslinking or polymerization of the block copolymer can be easily controlled.

Among them, glycidyl (meth)acrylate is more preferable, and glycidyl methacrylate is particularly preferable in consideration of further promoting the crosslinking reaction, ease of manufacture, and the like. That is, in a more preferred embodiment of the present invention, the reactive monomer having an epoxy group is at least one of glycidyl acrylate and glycidyl methacrylate. In a particularly preferred embodiment of the present invention, the reactive monomer having an epoxy group is glycidyl methacrylate.

The reactive monomer may be used singly or in combination of two or more types thereof. That is, the structural unit (A) derived from the reactive monomer may be a homopolymer type composed of a single type of reactive monomer or a copolymer type composed of two or more types of reactive monomers. The form of the structural unit (A) in the case of using two or more types may be a block copolymer or a random copolymer.

The hydrophilic monomer constituting the block copolymer has a swelling property upon contact with a body fluid or an aqueous solvent, and therefore imparts a lubricating property (surface lubricating property) to the medical device. Therefore, by introducing the structural unit (B) derived from a hydrophilic monomer into the block copolymer, the lubricating property (surface lubricating property) of the medical device is improved, and friction when the medical device comes into contact with a lumen wall such as a blood vessel wall can be reduced.

The hydrophilic monomer constituting the block copolymer is not particularly limited as long as it has the above characteristics, and a known compound can be used. Examples thereof include acrylamide and derivatives thereof, vinylpyrrolidone, acrylic acid and methacrylic acid and derivatives thereof, polyethylene glycol acrylate and derivatives thereof, a monomer having a sugar or a phospholipid in a side chain and a water-soluble monomer such as maleic anhydride. More specifically, examples thereof include acrylic acid, methacrylic acid, N-methylacrylamide, N,N-dimethylacrylamide (DMAA), acrylamide, acryloylmorpholine, N,N-dimethylaminoethyl acrylate, N-vinylpyrrolidone, 2-methacryloyloxyethyl phosphorylcholine, 2-methacryloyloxyethyl-D-glycoside, 2-methacryloyloxyethyl-D-mannoside, vinyl methyl ether, 2-hydroxyethyl (meth)acrylate, 4-hydroxybutyl (meth)acrylate, 2-hydroxypropyl (meth)acrylate, 2-hydroxybutyl (meth)acrylate, 6-hydroxyhexyl (meth)acrylate, 1,4-cyclohexanedimethanol mono(meth)acrylate, 1-chloro-2-hydroxypropyl (meth)acrylate, diethylene glycol mono(meth)acrylate, 1,6-hexanediol mono(meth)acrylate, pentaerythritol tri(meth)acrylate, dipentaerythritol penta(meth)acrylate, neopentyl glycol mono(meth)acrylate, trimethylolpropane di(meth)acrylate, trimethylolethane di(meth)acrylate, 2-hydroxy-3-phenyloxypropyl (meth)acrylate, 4-hydroxycyclohexyl (meth)acrylate, 2-hydroxy-3-phenyloxy (meth)acrylate, 4-hydroxycyclohexyl(meth)acrylate, cyclohexanedimethanol mono(meth)acrylate, poly(ethylene glycol) methyl ether acrylate, and poly(ethylene glycol) methyl ether methacrylate. From the viewpoint of imparting excellent lubricating property, ease of synthesis, and operability, the hydrophilic monomer preferably contains at least one selected from the group consisting of N,N-dimethylacrylamide, acrylamide, 2-hydroxyethyl methacrylate, and N-vinylpyrrolidone.

Among them, N,N-dimethylacrylamide, acrylamide, or 2-hydroxyethyl methacrylate is more preferable, and N,N-dimethylacrylamide is particularly preferable in consideration of imparting excellent lubricating property, ease of synthesis, and operability. That is, in a more preferred embodiment of the present invention, the hydrophilic monomer is at least one selected from the group consisting of N,N-dimethylacrylamide, acrylamide, and 2-hydroxyethyl methacrylate. Furthermore, in a particularly preferred form of the present invention, the hydrophilic monomer is N,N-dimethylacrylamide.

The hydrophilic monomer may be used singly or in combination of two or more types thereof. That is, the structural unit (B) derived from the hydrophilic monomer may be a homopolymer type composed of one type of hydrophilic monomer alone or a copolymer type composed of two or more types of the hydrophilic monomers. The form of the structural unit (B) in the case of using two or more types may be a block copolymer or a random copolymer.

The block copolymer has the structural unit (A) derived from the reactive monomer and the structural unit (B) derived from the hydrophilic monomer. Here, a ratio between the structural unit (A) and the structural unit (B) is not particularly limited as long as the above effect is obtained. In consideration of a good lubricating property, a lubrication retaining property, strength of the cover layer, bondability to the base layer, and the like, the ratio of the structural unit (A) to the structural unit (B) (molar ratio of the structural unit (A) : the structural unit (B)) is preferably 1 : 2 to 1 : 100, more preferably 1 : 2 to 1 : 50, still more preferably 1 : 5 to 1 : 50, and particularly preferably 1 : 10 to 1 : 30. Within such a range, the surface lubricating layer can sufficiently exhibit a lubricating property by the structural unit (B), and can exhibit sufficient cover layer strength, bondability to the base layer, and durability by the structural unit (A). The molar ratio of the structural unit (A) : the structural unit (B) can be controlled by adjusting the charging ratio (molar ratio) of each monomer in the manufacture stage of the block copolymer. Therefore, the charging ratio (molar ratio) between the reactive monomer having an epoxy group and the hydrophilic monomer in the manufacture stage of the block copolymer is preferably 1 : 2 to 1 : 100, more preferably 1 : 2 to 1 : 50, still more preferably 1 : 5 to 1 : 50, and particularly preferably 1 : 10 to 1 : 30. Note that the molar ratio of the structural unit (A) : the structural unit (B) can be confirmed, for example, by performing NMR measurement (¹H-NMR measurement, ¹³C-NMR measurement, etc.) on the copolymer.

The block copolymer according to the present invention essentially includes the structural unit (A) and the structural unit (B), but may have other structural units in addition to these structural units. When the block copolymer has other structural units, examples of the other structural units include adipic acid, glutaric acid, triethylene glycol, and tripropylene glycol. The monomers constituting other structural units may be used singly, or may be used in combination of two or more types thereof. That is, the other structural unit may be a homopolymer type composed of one type of structural unit alone or a copolymer type composed of two or more types of structural units. In a case where two or more types of monomers constituting the other structural unit are used, the segment constituted by the monomers may be in the form of a block copolymer, a random copolymer, or an alternating copolymer.

In a case where the block copolymer according to the present invention has other structural units, the content of the other structural units is preferably more than 0 mol% and 5 mol% or less with respect to all the structural units constituting the block copolymer. That is, in the block copolymer according to the present invention, when the total of all structural units constituting the block copolymer is 100 mol%, the total of the contents of the structural unit (A) and the structural unit (B) is preferably 95 mol% or more (upper limit: less than 100 mol%). More preferably, the block copolymer according to the present invention is substantially composed of the structural unit (A) and the structural unit (B) (the content of other structural units = more than 0 mol% and less than 5 mol%). In this form, the block copolymer according to the present invention can achieve durability by the structural unit (A) and the lubricating property (surface lubricating property) by the structural unit (B) in a well-balanced manner. Preferably, the block copolymer according to the present invention does not include the other structural unit (content of other structural unit = 0 mol%).

The composition of each structural unit (structural units (A) and (B) and other structural units) can be measured by a known method. For example, the composition (molar ratio) of the structural unit can be measured by measuring the integral ratio of the intensity of each signal in the ¹H-NMR spectrum of the block copolymer solution.

In an embodiment of the present invention, the block copolymer according to the present invention is substantially composed of the structural unit (A) derived from at least one reactive monomer selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether, and a structural unit (B) derived from at least one hydrophilic monomer selected from the group consisting of N,N-dimethylacrylamide, acrylamide, 2-hydroxyethyl methacrylate, and N-vinylpyrrolidone, or composed only of the structural unit (A) and the structural unit (B).

In an embodiment of the present invention, the block copolymer according to the present invention is substantially composed of the structural unit (A) derived from a reactive monomer of at least one of glycidyl acrylate and glycidyl methacrylate, and the structural unit (B) derived from at least one hydrophilic monomer selected from the group consisting of N,N-dimethylacrylamide, acrylamide, and 2-hydroxyethyl methacrylate, or composed only of the structural unit (A) and the structural unit (B) .

In an embodiment of the present invention, the block copolymer according to the present invention is substantially composed of the structural unit (A) derived from glycidyl methacrylate (reactive monomer having an epoxy group) and the structural unit (B) derived from N,N-dimethylacrylamide (hydrophilic monomer), or composed only of the structural unit (A) and the structural unit (B).

The weight average molecular weight of the block copolymer is preferably 10,000 to 10,000,000 from the viewpoint of solubility. The weight average molecular weight of the block copolymer is more preferably 100,000 to 5,000,000 from the viewpoint of ease of preparation of a coating liquid. In the present specification, as the "weight average molecular weight", a value measured by gel permeation chromatography (Gel Permeation Chromatography, GPC) using polystyrene as a reference material is adopted.

The method of manufacturing the block copolymer is not particularly limited, and the block copolymer can be produced by applying a conventionally known polymerization method such as a living radical polymerization method, a polymerization method using a macro initiator, or a polycondensation method. Among them, a living radical polymerization method or a polymerization method using a macro initiator is preferably used because it is easy to control the molecular weight and molecular weight distribution of the structural unit (moiety) derived from a reactive monomer and the structural unit (moiety) derived from a hydrophilic monomer. The living radical polymerization method is not particularly limited, but for example, methods described in JP H11-263819 A, JP 2002-145971 A, JP 2006-316169 A, and the like, an atom transfer radical polymerization (ATRP) method, and the like can be applied in the same manner or appropriately modified. In the polymerization method using a macro initiator, for example, a macro initiator having a reactive moiety having a reactive functional group and a radical polymerizable group such as a peroxide group is produced, and then a monomer for forming a hydrophilic moiety is polymerized with the macro initiator, whereby a block copolymer having a hydrophilic moiety and a reactive moiety can be produced.

The block copolymer after polymerization is preferably purified by a general purification method such as a reprecipitation method, a dialysis method, an ultrafiltration method, or an extraction method.

### (Silicone compound)

The silicone compound contained in the surface lubricating layer is not particularly limited, but may be reactive silicone, non-reactive silicone, or a combination thereof.

From the viewpoint of forming a uniform coating without phase separation from the block copolymer constituting the surface lubricating layer and stably obtaining slidability (surface lubricating property), the silicone compound preferably contains reactive silicone. By forming a uniform coating in this manner, adhesiveness (stickiness) in a drying process after wetting is more easily suppressed. When the reactive silicone is contained together with the block copolymer, these undergo a crosslinking reaction to form a network. As a result, it is presumed that the mobility of the block copolymer can be reduced, and the adhesiveness (stickiness) in the drying process after wetting is more easily suppressed. Furthermore, the network of the block copolymer and the reactive silicone formed by the crosslinking reaction is likely to swell in a wet state, so that excellent surface lubricating property can be obtained, and such excellent surface lubricating property can be obtained over a long period of time.

Here, the "reactive silicone" indicates a silicone compound having a reactive functional group contained in the block copolymer, for example, a reactive group capable of reacting with an epoxy group or the like (which may be a glycidyl group).

Therefore, in the present specification, "the surface lubricating layer contains a reactive silicone (as a silicone compound)" includes not only a form in which the surface lubricating layer contains an unreacted reactive silicone but also a form in which the surface lubricating layer further contains a reaction product of a reactive silicone and a block copolymer in addition to the reactive silicone. The "surface lubricating layer contains reactive silicone (as silicone compound) and block copolymer" includes not only a form in which the surface lubricating layer contains an unreacted reactive silicone and an unreacted block copolymer but also a form in which the surface lubricating layer contains a reaction product of a reactive silicone and a block copolymer. Here, the reaction rate between the reactive silicone and the block copolymer need not be 100%. That is, the surface lubricating layer may further contain at least one of unreacted reactive silicone and an unreacted block copolymer in addition to the reaction product.

The reactive group of the reactive silicone is not particularly limited, and examples thereof include a hydroxyl group (-OH), a carboxyl group, an amino group (-NH₂), an epoxy group, an ethylenically unsaturated group, and an alkoxysilyl group. Therefore, in one embodiment, the reactive silicone preferably has at least one selected from the group consisting of a hydroxyl group (-OH), a carboxyl group, an amino group, an epoxy group, an ethylenically unsaturated group, and an alkoxysilyl group. More preferably, the reactive silicone has at least one selected from the group consisting of a hydroxyl group, a carboxyl group, an amino group, and an epoxy group. Particularly preferably, the reactive silicone has an amino group and/or an epoxy group. The epoxy group may be contained in the form of a glycidyl group.

The reactive silicone is not particularly limited as long as it is a silicone having the reactive group, and examples thereof include amino-modified silicone, epoxy-modified silicone, carboxy-modified silicone, (meth)acrylic-modified ((meth)acryloyl-modified) silicone, carbinol-modified silicone, silanol-modified silicone, and the like. Among them, the reactive silicone is preferably an amino-modified silicone and/or an epoxy-modified silicone because of excellent reactivity with an epoxy group contained in the block copolymer. The reactive silicone can be used singly or in combination of two or more types thereof. In a case where two or more types are used, the content of the reactive silicone represents the total amount thereof.

In one embodiment, the silicone compound constituting the surface lubricating layer may be a reactive silicone. That is, the silicone compound contained in the surface lubricating layer may be substantially composed of only reactive silicone. Here, the "form in which the silicone compound contained in the surface lubricating layer is substantially composed of only reactive silicone" is a form in which a silicone compound other than reactive silicone (that is, non-reactive silicone) is not contained in the surface lubricating layer at least intentionally. Therefore, a surface lubricating layer inevitably containing a trace amount of a silicone compound other than reactive silicone derived from a raw material, a production method, or the like is included in the above form. In other words, the above form is a form in which the silicone compound contained in the surface lubricating layer does not contain a silicone compound other than reactive silicone.

In a preferred embodiment, the silicone compound constituting the surface lubricating layer further contains a non-reactive silicone in addition to the reactive silicone. That is, the silicone compound contained in the surface lubricating layer preferably contains reactive silicone and non-reactive silicone. According to such a form, an effect of suppressing adhesiveness (stickiness) in a drying process after wetting is more easily obtained, and slidability in a wet state is also excellent. Here, the "non-reactive silicone" indicates a silicone compound having no reactive group.

The non-reactive silicone is not particularly limited as long as it is a silicone having no reactive group, and examples thereof include dimethyl silicone, methyl phenyl silicone, and methyl hydrogen silicone. Among them, dimethyl silicone is preferable as the non-reactive silicone. The non-reactive silicone can be used singly or in combination of two or more types thereof. In a case where two or more types are used, the content of the non-reactive silicone represents the total amount thereof.

As the silicone compound, a commercially available product may be used, or a synthetic product may be used. As commercially available products of the reactive silicone, KF-393, KF-859, KF-860, KF-864, KF-865, KF-868, KF-880, KF-8002, KF-8004, KF-8005, KF-8008, KF-8012, X-22-343, KF-101, KF-1001, X-22-2000, X-22-4039, X-22-4015, X-22-3701E, X-22-4741, KF-1002, X-22-163, X-22-164 (manufactured by Shin-Etsu Chemical Co., Ltd.) and the like may be used. As commercially available products of the non-reactive silicone, SH-200, SH510, SH550, SH710 (manufactured by Dow Corning Toray Co., Ltd.), KF-96, KF-96H, KF-965, KF-968, KF-50, KF-53, KF-54 (manufactured by Shin-Etsu Chemical Co., Ltd.), and the like may be used.

In addition, as a synthesis method, a known method may be used as it is, or a synthesis method of a silicone compound may be appropriately modified and used. Examples of the method of synthesizing the reactive silicone include a method of reacting an amino group-containing silane with a silanol group-containing polydiorganosiloxane, and a method of reacting an epoxy group-containing silane with a silanol group-containing polydiorganosiloxane. The reaction between the silane compound having a reactive group and the polydiorganosiloxane containing a silanol group may be performed by using a solvent as necessary and stirring and heating these compounds. At this time, the reaction is preferably performed so that the amino group-containing silane/epoxy group-containing silane is 1 to 5% by mass and the silanol group-containing polydiorganosiloxane is 95 to 99% by mass (100% by mass in total).

Examples of the amino group-containing silane include γ-aminopropyltrimethoxysilane, γ-aminopropylmethyldimethoxysilane, γ-aminopropyltriethoxysilane, γ-aminopropylmethyldiethoxysilane, N-(β-aminoethyl)aminomethyltrimethoxysilane, γ-(β-aminoethyl)aminopropyltrimethoxysilane, γ-(N-(β-aminoethyl) amino)propylmethyldimethoxysilane, N-(β-aminoethyl)aminomethyltributoxysilane, and γ-(N-(β-(N-(β-aminoethyl) amino) ethyl) amino)propyltrimethoxysilane.

Examples of the epoxy group-containing silane include γ-glycidoxyproltrimethoxysilane, γ-glycidoxypromethyldimethoxysilane, β-(3,4-epoxycyclohexyl) ethyltrimethoxysilane, β-(3,4-epoxycyclohexyl) ethylmethyldimethoxysilane, β- (3,4-epoxycyclohexyl) ethyltriethoxysilane, and β-(3,4-epoxycyclohexyl) ethylmethyldiethoxysilane.

Examples of the organic group bonded to the silicon atom of the silanol group contained in the polydiorganosiloxane containing a silanol group include an alkyl group such as a methyl group, a phenyl group, and a vinyl group. From the viewpoint of ease of synthesis of polydiorganosiloxane, the organic group is preferably a methyl group or a phenyl group, and more preferably a methyl group. Specific examples of the polydiorganosiloxane containing a silanol group include polydimethylsiloxane in which one end is blocked with a silanol group and the other end is blocked with a trimethylsilyl group, polydimethylsiloxane in which both ends are blocked with a silanol group, and polymethylphenylsiloxane in which both ends are blocked with a silanol group.

The content of the silicone compound in the surface lubricating layer is not particularly limited as long as it is an amount that can satisfy each range of the water contact angles θ1 and θ2 (preferably, also the water contact angle θ3).

The mass ratio of the silicone compound and the block copolymer (silicone compound : block copolymer) in the surface lubricating layer is preferably 0.1 to 1 : 1. That is, the surface lubricating layer preferably contains a silicone compound and a block copolymer at a mass ratio of 0.1 to 1 : 1 (silicone compound : block copolymer). Furthermore, the mass ratio of the silicone compound and the block copolymer in the surface lubricating layer is more preferably more than 0.1 and less than 1 : 1, and particularly preferably more than 0.1 and 0.8 or less : 1. By setting the mass ratio of the silicone compound and the block copolymer in the surface lubricating layer within the above range, it is easy to control the water contact angles θ1 and θ2 of the surface lubricating layer within a predetermined range. In addition, the water contact angle θ3 can be set within the above preferable range. Furthermore, since the ratio of the block copolymer exposed on the surface of the surface lubricating layer can be appropriately reduced, the generation of adhesiveness (stickiness) in the drying process by the block copolymer can be more easily suppressed.

In the present specification, in a case where the silicone compound contains non-reactive silicone in addition to reactive silicone, the "content of the silicone compound" indicates the total amount thereof. Furthermore, the above "content of silicone compound" indicates the total mass of silicone compound components (total mass of an unreacted component and a reacted component with the block copolymer). In the present specification, the content of the reactive silicone indicates the total mass of the unreacted component and the reacted component with the block copolymer.

In a case where the surface lubricating layer contains a reactive silicone as the silicone compound, the mass ratio of the reactive silicone to the block copolymer is not particularly limited, but the mass ratio of the reactive silicone to the block copolymer (reactive silicone : block copolymer) in the surface lubricating layer is preferably 0.01 to 1 : 1, more preferably 0.03 to 0.9 : 1, and particularly preferably 0.04 to 0.8 : 1. When the mass ratio is set as described above, phase separation from the block copolymer is more effectively suppressed, and a uniform coating is easily formed. In addition, while a sufficient lubricating property (slidability) of the surface lubricating layer is secured, crosslinking of the block copolymer is sufficiently progressed and not excessively progressed (crosslinking can be moderately promoted). Therefore, the lubricating property and durability of the resulting surface lubricating layer can be sufficiently exhibited.

When the surface lubricating layer contains reactive silicone and non-reactive silicone as the silicone compound, the mass ratio thereof is not particularly limited, but the mass ratio of the reactive silicone and the non-reactive silicone (reactive silicone : non-reactive silicone) in the surface lubricating layer is preferably 0.01 to 1 : 1, more preferably 0.03 to 1 : 1, still more preferably 0.05 to 1 : 1, still more preferably 0.1 to 1 : 1, particularly preferably 0.5 to 0.8 : 1, and most preferably 0.6 to 0.7 : 1. When the mass ratio is set as described above, phase separation from the block copolymer is more effectively suppressed, and a uniform coating is easily formed. In addition, while the sufficient lubricating property (slidability) of the surface lubricating layer is secured, crosslinking of the block copolymer is sufficiently progressed and not excessively progressed (crosslinking can be moderately promoted). Therefore, the lubricating property and durability of the resulting surface lubricating layer can be sufficiently exhibited.

### (Other components)

The surface lubricating layer may contain other components in addition to the block copolymer and the silicone compound. The other components are not particularly limited, and are appropriately selected according to the use of the medical device, for example.

### (Average tension (sliding resistance value))

From the viewpoint of obtaining excellent surface lubricating property and suppressing adhesiveness (stickiness) in a drying process after wetting, the surface lubricating layer of the medical device according to the present invention preferably has an average tension of less than 550 gf, more preferably less than 500 gf, still more preferably less than 400 gf, and particularly preferably less than 300 gf during 40 times of sliding in the dry process after wetting. On the other hand, the lower limit is not particularly limited, but is about 100 gf. As a specific method of measuring the average tension, the method described in the section [Evaluation of sliding resistance in drying process after wetting] described in Examples is adopted.

Furthermore, from the viewpoint of improving operability during a procedure, the surface lubricating layer of the medical device according to the present invention preferably has an average tension of 200 gf or less, more preferably 150 gf or less, still more preferably less than 100 gf, particularly preferably less than 50 gf, and most preferably 40 gf or less during 40 times of sliding in a wet state. On the other hand, the lower limit is not particularly limited, but is about 10 gf. As a specific method of measuring the average tension, the method described in the section [Evaluation of sliding resistance in wet state] described in Examples is adopted.

### [Method of manufacturing medical device]

The surface lubricating layer of the medical device may be formed by using any method as long as a coating containing a block copolymer and a silicone compound can be formed on the base layer, but is preferably formed by applying a coating liquid containing a block copolymer, a silicone compound, and a solvent onto the base layer.

Therefore, another aspect of the present invention provides a method of manufacturing a medical device including a base layer and a surface lubricating layer supported on at least a part of the base layer, the method including: preparing a coating liquid containing a block copolymer having a structural unit (A) derived from a reactive monomer having an epoxy group and a structural unit (B) derived from a hydrophilic monomer, a silicone compound, and a solvent; and applying the coating liquid onto the base layer. Hereinafter, the method of manufacturing the medical device having the above configuration will also be referred to as "manufacturing method according to the present invention" or "manufacturing method".

A method of manufacturing a medical device according to the present invention includes preparing a coating liquid containing a block copolymer having a structural unit (A) derived from a reactive monomer having an epoxy group and a structural unit (B) derived from a hydrophilic monomer, a silicone compound, and a solvent ((I) preparation step); and applying the coating liquid onto the base layer ((II) application step). As described above, by using a solution containing a silicone compound together with a block copolymer as a coating liquid, a medical device having a surface lubricating layer exhibiting excellent lubrication retaining property even in a drying process after wetting is obtained.

A medical device manufactured by using the method according to the present invention has a configuration in which a coating liquid containing a block copolymer for forming a surface lubricating layer and a silicone compound is applied onto a base layer, so that the surface lubricating layer is supported on the base layer. In the coating liquid, the block copolymer and the silicone compound are dissolved in a solvent (The block copolymer and the silicone compound are uniformly mixed in the coating liquid). Therefore, the block copolymer and the silicone compound can form a uniform surface lubricating layer without forming a phase separation structure.

Therefore, the surface lubricating layer of the medical device manufactured by using the manufacturing method can easily control the water contact angle θ1 to 104° or more and the water contact angle θ2 to 102° or less. In addition, it is easy to achieve the water contact angle θ3 exceeding 102°. Furthermore, according to the manufacturing method, it is easy to control each of the water contact angles θ1, θ2, and θ3 described in the section [Medical device] within a preferable range.

In the method of manufacturing a medical device according to the present invention, the terms such as the water contact angles θ1, θ2, and θ3, the block copolymer, and the silicone compound are the same as those described in the section [Medical device], and thus the description thereof will be omitted here.

In the method of manufacturing a medical device according to the present invention, after the step (II), steps such as a step of performing drying and/or heat treatment ((III) drying/heat treatment step) and a step of cleaning ((IV) cleaning step) may be further performed as necessary. Among them, it is preferable to further perform at least the (III) drying/heat treatment step after the step (II). Hereinafter, each of the steps (I) to (IV) will be described.

### (I) Preparation step

In this step, a coating liquid containing a block copolymer, a silicone compound, and a solvent is prepared. Here, in this step, the coating liquid may be prepared by mixing a block copolymer, a silicone compound, and a solvent to prepare a coating liquid. Alternatively, a coating liquid containing a block copolymer, a silicone compound, and a solvent may be purchased, and the coating liquid may be used.

Hereinafter, preferred embodiments of preparing a coating liquid by mixing a block copolymer, a silicone compound, and a solvent to prepare a coating liquid will be described in detail.

### (Preparation of coating liquid)

A coating liquid is prepared by using the block copolymer, the silicone compound, and the solvent. The order of addition and the method of addition of the block copolymer, the silicone compound, and the solvent are not particularly limited. The above components may be added collectively or separately, stepwise or continuously.

In addition, a method of mixing the components is not particularly limited, and a known method may be used. Examples of the method of preparing the coating liquid include a method of sequentially adding a silicone compound and a block copolymer to a solvent, a method of sequentially adding a block copolymer and a silicone compound to a solvent, and a method of collectively adding a silicone compound and a block copolymer to a solvent.

From the viewpoint of easily preparing a uniform solution (coating liquid), it is preferable to sequentially add a silicone compound and a block copolymer to a solvent. If necessary, the addition may be performed while stirring. More specifically, it is preferable to mix the solvent and the silicone compound (preferably, the silicone compound) to obtain a solution of the silicone compound, and then further mix the block copolymer. By adopting such a form, a phase separation structure between the block copolymer and the silicone compound is more hardly formed, and a more uniform surface lubricating layer can be formed. As a result, the generation of adhesiveness (stickiness) in the drying process after wetting can be further suppressed.

The solvent used for preparing the coating liquid is not particularly limited as long as it can dissolve the block copolymer and the silicone compound (and other components, if used), and is appropriately selected according to the type of the block copolymer and the silicone compound (and other components, if used). Specific examples of the solvent of the coating liquid include alcohol solvents such as methanol, ethanol, isopropyl alcohol, butanol, and ethylene glycol; ketone-based solvents such as acetone, methyl ethyl ketone, and cyclohexanone; ester solvents such as ethyl acetate and butyl acetate; ether-based solvents such as tetrahydrofuran (THF), butyl ether, and dioxane; aliphatic hydrocarbon-based solvents such as hexane and heptane; aromatic hydrocarbon-based solvents such as benzene and toluene; and dimethyl sulfoxide (DMSO), and N,N-dimethylformamide (DMF). These may be used singly or in mixture of two or more types thereof (in a form of a mixed solvent).

Among them, from the viewpoint of excellent solubility of the block copolymer and the silicone compound (in particular, reactive silicones), the solvent preferably contains at least one selected from the group consisting of methanol, ethanol, isopropyl alcohol, butanol, and ethylene glycol; acetone, methyl ethyl ketone, and cyclohexanone; ethyl acetate and butyl acetate; tetrahydrofuran (THF), butyl ether, and dioxane; hexane and heptane; benzene and toluene; dimethyl sulfoxide (DMSO); and N,N-dimethylformamide (DMF), more preferably contains at least one selected from the group consisting of acetone, methyl ethyl ketone, and cyclohexanone; and tetrahydrofuran, butyl ether, and dioxane, still more preferably contains acetone and/or tetrahydrofuran, and particularly preferably contains tetrahydrofuran.

The concentration of the block copolymer in the coating liquid is not particularly limited. From the viewpoint of further improving coatability, lubricating property, and durability of the surface lubricating layer, the concentration of the block copolymer in the coating liquid is preferably 0.5 to 20% by mass, more preferably 1 to 15% by mass, and particularly preferably 3 to 10% by mass. When the concentration of the block copolymer is within the above range, the lubricating property and durability of the resulting surface lubricating layer can be sufficiently exhibited. In addition, a uniform surface lubricating layer having a desired thickness can be easily obtained by one coating, and the viscosity of the solution falls within an appropriate range, which is preferable in terms of operability (ease of coating, for example) and production efficiency. However, even if it is out of the above range, it can be sufficiently used as long as it does not affect the operation and effect of the present invention.

The concentration of the silicone compound in the coating liquid is not particularly limited as long as the concentration can satisfy each range of the water contact angles θ1 and θ2 (preferably, the water contact angle θ3). From such a viewpoint, the concentration of the silicone compound in the coating liquid is preferably 0.01 to 15% by mass more preferably 0.1 to 10% by mass, still more preferably 0.5 to 5% by mass, and particularly preferably 0.5% by mass or more and less than 5% by mass. When the concentration of the silicone compound is within the above range, adhesiveness (stickiness) in a drying process after wetting can be effectively suppressed. However, even if it is out of the above range, it can be sufficiently used as long as it does not affect the operation and effect of the present invention.

Furthermore, the preferable mixing ratio (mass ratio) of the block copolymer and the silicone compound in the coating liquid is the same as the preferable content ratio (mass ratio) of the block copolymer and the silicone compound present in the surface lubricating layer. When the mass ratio of the block copolymer and the silicone compound in the coating liquid is in the above range, it is easy to control the water contact angles θ1 and θ2 of the surface lubricating layer within a predetermined range. In addition, the water contact angle θ3 can be set within the above preferable range. Furthermore, the durability and the lubricating property of the surface lubricating layer can be sufficiently exhibited. In a case where a reactive silicone is used as the silicone compound, a preferable mixing ratio (mass ratio) of the reactive silicone and the block copolymer in the coating liquid is the same as a preferable mass ratio of the reactive silicone and the block copolymer in the surface lubricating layer.

When a reactive silicone and a non-reactive silicone are used as the silicone compound, a preferable mixing ratio (mass ratio) of the reactive silicone and the non-reactive silicone in the coating liquid is the same as a preferable mass ratio of the reactive silicone and the non-reactive silicone in the surface lubricating layer. When the mass ratio of the reactive silicone and the non-reactive silicone in the coating liquid is in the above range, the crosslinking of the block copolymer is allowed to proceed sufficiently but is prevented from proceeding excessively (the crosslinking can be appropriately promoted) while sufficiently securing the lubricating property (slidability) of the surface lubricating layer. Therefore, the lubricating property and durability of the resulting surface lubricating layer can be sufficiently exhibited.

### (II) Coating step

In this step, the coating liquid prepared in the (I) preparation step is applied onto the base layer to form a coating film (coating layer) on the base layer.

The base layer may be made of any material, and examples thereof include a metal material, a polymer material, and ceramics. Specific examples of these materials are the same as those described in the above section <Base layer (base material)>, and thus detailed description thereof will be omitted here.

The method of applying the coating liquid to the surface of the base layer is not particularly limited, and conventionally known methods such as a coating/printing method, an immersion method (dipping method, dip coating method), a spraying method, a spin coating method, a mixed solution impregnation sponge coating method, a bar coating method, a die coating method, a reverse coating method, a comma coating method, a gravure coating method, and a doctor knife method can be applied. Among them, an immersion method (dipping method, dip coating method) is preferably used.

When a surface lubricating layer is formed on a thin and narrow inner surface of a catheter, a guide wire, an injection needle, or the like, the base layer may be immersed in a coating liquid to depressurize the inside of the system to defoam. The solution can be quickly permeated into the thin and narrow inner surface to promote the formation of the surface lubricating layer by reducing the pressure to defoam.

In a case where the surface lubricating layer is formed only on a part of the base layer, the surface lubricating layer can be formed on a desired surface portion of the base layer by immersing only a part of the base layer in a coating liquid and coating the part of the base layer with the coating liquid.

When it is difficult to immerse only a part of the base layer in the coating liquid, a surface portion of the base layer on which the surface lubricating layer does not need to be formed is protected (covered or the like) in advance with an appropriate member or material that can be attached/detached (donned/doffed), the base layer is immersed in the coating liquid to coat the base layer with the coating liquid, then a protective member (material) of the surface portion of the base layer on which the surface lubricating layer does not need to be formed is removed, and then the protective member is reacted by heat treatment or the like, whereby the surface lubricating layer can be formed on a desired surface portion of the base layer. However, the present invention is not limited to these forming methods, and the surface lubricating layer can be formed by appropriately using a conventionally known method. For example, when it is difficult to immerse only a part of the base layer in the coating liquid, another coating method (for example, a method of applying the coating liquid to a predetermined surface portion of the medical device using a spray device or a coating device such as a bar coater, a die coater, a reverse coater, a comma coater, a gravure coater, a spray coater, or a doctor knife) may be applied instead of the immersion method. Note that in a case where both the outer surface and the inner surface of the cylindrical tool need to have a surface lubricating layer due to the structure of the medical device, an immersion method (dipping method) is preferably used because both the outer surface and the inner surface can be coated at a time.

The coating amount of the coating liquid is preferably such an amount that the thickness (dry film thickness) of the resulting coating (surface lubricating layer) is 0.1 to 10 µm, more preferably such an amount that the thickness is 0.3 to 5 µm, and still more preferably such an amount that the thickness is 0.5 to 3 µm. When the coating amount is such an amount that the thickness of the coating (surface lubricating layer) is 0.1 µm or more, the durability of the resulting coating (surface lubricating layer) can be sufficiently achieved. When the coating amount is such an amount that the thickness of the coating (surface lubricating layer) is 10 µm or less, the surface of the coating (surface lubricating layer) is less likely to be sticky, and handling at the time of manufacturing becomes easier.

### (III) Drying/heat treatment step

In the method of manufacturing a medical device according to the present invention, if necessary, it is preferable to perform a heat treatment step after forming a coating film (cover layer) by applying a coating liquid on the base layer in the (II) coating step. By performing the heat treatment step, not only the solvent can be easily removed, but also a stronger surface lubricating layer can be formed. In addition, it becomes easier to control the water contact angles θ1 and θ2 (furthermore, the water contact angle θ3).

The condition during the heat treatment is not particularly limited as long as it is a condition under which a surface lubricating layer containing the block copolymer and the silicone compound and satisfying each range of the water contact angles θ1 and θ2 (preferably, also the water contact angle θ3) can be formed on the base layer.

The temperature of the heat treatment is not particularly limited, but is preferably 50 to 200°C, and more preferably 100 to 180°C. By maintaining the temperature at such a temperature (heat treatment), it is easy to control the water contact angles θ1 and θ2 (furthermore, the water contact angle θ3) of the surface lubricating layer to be formed within a predetermined range. In addition, by maintaining the temperature at the above temperature, a strong cover layer (surface lubricating layer) is formed. Note that the temperature may be changed during the heat treatment.

The time for the heat treatment is also not particularly limited, but is preferably 15 minutes to 20 hours, and more preferably 30 minutes to 12 hours. By setting such a time, the water contact angles θ1 and θ2 (furthermore, the water contact angle θ3) of the surface lubricating layer to be formed can be easily controlled within a predetermined range. In addition, since the crosslinking reaction in the block copolymer is effectively promoted and a strong cover layer (surface lubricating layer) is formed, a high lubricating property (surface lubricating property) can be maintained for a longer period of time.

A drying step may be performed before the heat treatment step. In the drying step, since the solvent is removed to some extent, the subsequent heat treatment step can be more efficiently advanced. The temperature of the drying step is not particularly limited, but the drying step is preferably performed at 10°C or more and less than 50°C, and more preferably performed at 20 to 30°C. The drying time is also not particularly limited, but is preferably 15 minutes to 20 hours, and more preferably 30 minutes to 12 hours. The pressure condition during drying is not limited at all, and the drying may be performed under normal pressure (atmospheric pressure), or may be performed under pressure or reduced pressure.

As means (device) for performing the heat treatment step and the drying step, for example, an oven and a vacuum dryer may be used, but in the case of natural drying, drying means (device) is particularly unnecessary.

### <Application of medical device>

Examples of the medical device according to the present invention include a device used in contact with body fluid, blood, or the like, and the device has a lubricating property on a surface thereof in an aqueous liquid such as body fluid or physiological saline, and can improve operability and reduce damage to tissue mucosa. Specific examples of the medical device according to the present invention include, but are not limited to, a catheter, a guide wire, and an indwelling needle used in a blood vessel.

### Examples

The effects of the present invention will be described with reference to the following Examples and Comparative Examples. However, the technical scope of the present invention is not limited only to the following Examples. Note that, in the following Examples, unless otherwise specified, an operation was performed at room temperature (25°C). Unless otherwise specified, "%" and "parts" mean "% by mass" and "parts by mass", respectively.

### Synthesis Example 1

The following reaction was allowed to proceed, and a block copolymer (1) was manufactured.

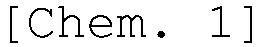

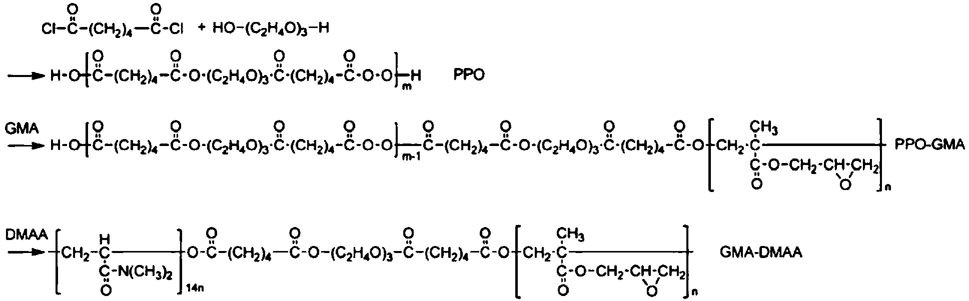

After 29.7 g of triethylene glycol was added dropwise to 72.3 g of adipic acid dichloride at 50°C, hydrochloric acid was removed at 50°C for 3 hours under reduced pressure to obtain an oligoester. Next, 4.5 g of methyl ethyl ketone was added to 22.5 g of the obtained oligoester, and the mixture was added dropwise to a solution containing 5 g of sodium hydroxide, 6.93 g of 31% hydrogen peroxide, 0.44 g of dioctyl phosphate as a surfactant, and 120 g of water and reacted at -5°C for 20 minutes. The resulting product was repeatedly washed with water and methanol, and then, dried to obtain a poly peroxide (PPO) having a plurality of peroxide groups in a molecule.

Next, 0.5 g of this PPO and 9.5 g of glycidyl methacrylate (GMA) were polymerized in 30 g of benzene as a solvent while being stirred at 80°C for 2 hours under reduced pressure. The reaction product obtained after the polymerization was reprecipitated with diethyl ether to obtain polyglycidyl methacrylate having a plurality of peroxide groups in a molecule (PPO-GMA).

Subsequently, 1.0 g of the obtained PPO-GMA (corresponding to 7 mmol of GMA) was charged into 9.0 g of N,N-dimethylacrylamide (DMAA) and 90 g of dimethyl sulfoxide as a solvent, and the mixture was reacted at 80°C for 18 hours. The reaction product obtained after the reaction was reprecipitated with hexane and recovered to obtain a block copolymer (1) (structural unit (A) : structural unit (B) = GMA : DMAA = 1 : 14 (molar ratio)) having an epoxy group in the molecule and exhibiting lubricating property in a wet state. The block copolymer (1) thus obtained was analyzed by ¹H-NMR and ATR-IR, and it was confirmed that an epoxy group was present in the molecule. The weight average molecular weight (Mw) of the block copolymer (1) measured by gel permeation chromatography (GPC, polystyrene equivalent) was about 1.5 million.

### Example 1

The amino group-containing silicone (1) (KF-859 manufactured by Shin-Etsu Chemical Co., Ltd., functional group equivalent: 6,000 g/mol, reactive silicone (1)) was dissolved in tetrahydrofuran (THF) so that the final concentration in the coating liquid was 2.0% by mass (solution (1)). The block copolymer (1) synthesized in Synthesis Example 1 was added to and dissolved in the solution (1) so that the final concentration in the coating liquid was 5.0% by mass to prepare the coating liquid (1).

The SUS304 polishing rod (RGOS3-200 manufactured by Misumi Co., Ltd., outer diameter: 3.00 mm) was immersed in the coating liquid (1), pulled up at a pulling speed of 8 mm/min, and dried at room temperature (25°C) for 1 hour to remove THF, thereby forming the coating film (1) on the surface of the rod (rod (1)). Further, the rod (1) was stored in an oven at 130°C for 1 hour, and the coating film (1) was heat-treated (rod (1')). The rod (1') was cooled to room temperature (25°C) to prepare the sample (covered rod) (1) having a cover layer (surface lubricating layer) (dry film thickness: 1 µm) containing the block copolymer (1) and the amino group-containing silicone (1) (including the reactant) on the surface of the rod.

### Example 2

The sample (covered rod) (2) was prepared in the same manner as in Example 1 except that the final concentration of the amino group-containing silicone (1) in the coating liquid was changed to 4.0% by mass, the coating liquid (2) was prepared, and the coating film (2) was formed by using the coating liquid (2) in Example 1.

### Example 3

The sample (covered rod) (3) was prepared in the same manner as in Example 1, except that the coating liquid (3) was prepared by using the silicone compound (2) prepared as follows instead of the amino group-containing silicone (1) in Example 1, and the coating film (3) was formed by using the coating liquid (3):
First, polydimethylsiloxane having 3-aminopropyl groups at both ends (manufactured by Merck Corporation, number average molecular weight: 2,500, reactive silicone (2)), polydimethylsiloxane having glycidyl ether groups at both ends (manufactured by Merck Corporation, number average molecular weight: 800, reactive silicone (3)), and dimethylsilicone oil (KF-96 manufactured by Shin-Etsu Chemical Co., Ltd., non-reactive silicone) were mixed at a mass ratio of 35 : 5 : 60 (reactive silicone (2) : reactive silicone (3) : non-reactive silicone), and the resulting mixture was taken as the silicone compound (2).

The silicone compound (2) was dissolved in tetrahydrofuran (THF) to have a concentration of 30% by mass. Then, the silicone compound (2) was diluted with tetrahydrofuran (THF) to have a final concentration of 0.57% by mass (solution (3)). The block copolymer (1) synthesized in Synthesis Example 1 was added to and dissolved in the solution (3) so that the final concentration in the coating liquid was 5.0% by mass to prepare the coating liquid (3).

### Example 4

The sample (covered rod) (4) was prepared in the same manner as in Example 3 except that the final concentration of the silicone compound (2) in the coating liquid was changed to 1.1% by mass, the coating liquid (4) was prepared, and the coating film (4) was formed using the coating liquid (4) in Example 3.

### Comparative Example 1

The block copolymer (1) synthesized in Synthesis Example 1 was dissolved in tetrahydrofuran (THF) so that the final concentration thereof in the coating liquid was 5.0% by mass to prepare the comparative coating liquid (1).

The SUS304 polishing rod (RGOS3-200 manufactured by Misumi Co., Ltd., outer diameter: 3.00 mm) was immersed in the comparative coating liquid (1), pulled up at a pulling speed of 8 mm/min, and dried at room temperature (25°C) for 1 hour to remove THF, thereby forming the comparative coating film (1) on the surface of the rod (comparative rod (1)). Furthermore, the comparative rod (1) was stored in an oven at 130°C for 1 hour, and the comparative coating film (1) was heat-treated (comparative rod (1')). The comparative rod (1') was cooled to room temperature (25°C) to prepare the sample (comparative covered rod) (1) having a cover layer (surface lubricating layer) (dry film thickness: 1 µm) containing the block copolymer (1) on the rod surface.

### Comparative Example 2

The amino group-containing silicone (1) (KF-859 manufactured by Shin-Etsu Chemical Co., Ltd., functional group equivalent: 6,000 g/mol) was dissolved in tetrahydrofuran (THF) so that the final concentration thereof in the coating liquid was 4.0% by mass to prepare the comparative coating liquid (2).

The SUS304 polishing rod (RGOS3-200 manufactured by Misumi Co., Ltd., outer diameter: 3.00 mm) was immersed in the comparative coating liquid (2), pulled up at a pulling speed of 8 mm/min, and dried at room temperature (25°C) for 1 hour to remove THF, thereby forming the comparative coating film (2) on the surface of the rod (comparative rod (2)). Further, this comparative rod (2) was stored in an oven at 130°C for 1 hour, and the comparative coating film (2) was heat-treated (comparative rod (2)). The comparative rod (2) was cooled to room temperature (25°C) to prepare the sample (comparative covered rod) (2) having a cover layer (surface lubricating layer) (dry film thickness: 1 µm) containing an amino group-containing silicone (1) on the surface of the rod.

### Comparative Example 3

The sample (comparative covered rod) (3) was prepared in the same manner as in Comparative Example 2 except that the comparative coating liquid (3) was prepared by using the silicone compound (2) obtained in the same manner as in Example 3 instead of the amino group-containing silicone (1) in Comparative Example 2, and the comparative coating film (3) was formed by using the comparative coating liquid (3).

### [Measurement of contact angle]

By using the coating liquids (1) to (4) prepared in the above Examples 1 to 4 and the comparative coating liquids (1) to (3) prepared in the above Comparative Examples 1 to 3, a cover layer (surface lubricating layer) was formed in the same manner as in the preparation procedure of each of the rods, and the contact angles (θ1, θ2, and θ3) of the surface lubricating layer were measured according to the following method:
The coating liquid was stirred for 1 hour by using a stirrer, and then the SUS304 plate subjected to surface polishing was immersed in the coating liquid, pulled up at a pulling speed of 8 mm/min, and dried at room temperature (25°C) for 1 hour to remove THF, thereby forming a coating film. Next, the SUS plate on which the coating film was formed was stored in an oven at 130°C for 1 hour, and the coating film was heat-treated. Thereafter, the SUS plate on which the coating film was formed was cooled to room temperature (25°C) to obtain samples for contact angle measurement (plates (1) to (4) and comparative plates (1) to (3)) having a cover layer (surface lubricating layer) on the plate surface.

Purified water was filled in a dedicated syringe of a contact angle measuring device (manufactured by Asumi Giken Co., Ltd., device name: B100), and a 25G syringe needle was attached. The syringe was attached to the contact angle measuring device, and the sample for contact angle measurement obtained in the above procedure was set on a board surface. At this time, the discharge amount of the syringe was adjusted by using an image analysis function (pendant drop method) so that the amount of droplets was 1.0 µL. Thereafter, the board surface was raised to bring the sample surface into contact with the droplet, and then the board surface was lowered to transfer the droplet from the syringe needle onto the sample. The elapsed time at the time at which the droplet moved onto the sample was used as a reference (0 second), and the contact angle after the lapse of a specified time (5 s (θ1), 1 min (θ3), 5 min (θ2)) (contact angle at the time when the specified time has elapsed) was measured by using the tangent method. The measurement was performed under the conditions of room temperature (25°C) and a relative humidity of 50%RH. The obtained results are illustrated in Table 1 below. In the following Table 1, the block copolymer (hydrophilic block copolymer) (1) synthesized in the above Synthesis Example 1 is represented as "p(GMA-b-DMAA)". In addition, the mass ratio of the silicone compound and the block copolymer (silicone compound : block copolymer) is represented as "silicone compound/block copolymer mass ratio".

**[Table 1]**

| | Silicone compound | | Block copolymer | Silicone compound/block copolymer mass ratio | Contact angle [°] | | |
|---|---|---|---|---|---|---|---|
| | Type | Concentration in coating liquid [% by mass] | | | θ1 (after 5 seconds) | θ2 (after 5 minutes) | θ3 (after 1 minute) |
| Example 1 | Amino group-containing silicone (1) | 2.0 | p(GMA-b-DMAA) | 0.40:1 | 120.7 | 99.1 | 110.1 |
| Example 2 | Amino group-containing silicone (1) | 4.0 | p(GMA-b-DMAA) | 0.80:1 | 104.3 | 93.5 | 103.5 |
| Example 3 | Silicone compound (2) (mixture) | 0.57 | p(GMA-b-DMAA) | 0.11:1 | 124.6 | 94.8 | 112.7 |
| Example 4 | Silicone compound (2) (mixture) | 1.1 | p(GMA-b-DMAA) | 0.22:1 | 124.6 | 101.6 | 117.4 |
| Comparative Example 1 | - | - | p(GMA-b-DMAA) | - | 100.7 | 72.8 | 92.3 |
| Comparative Example 2 | Amino group-containing silicone (1) | 4.0 | - | - | 88.9 | 70.6 | 84.5 |
| Comparative Example 3 | Silicone compound (2) (mixture) | 4.0 | - | - | 100.1 | 76.0 | 92.4 |

### [Evaluation of sliding resistance in drying process after wetting]

For the samples (1) to (4) prepared in Examples 1 to 4 and the comparative samples (1) to (3) prepared in Comparative Examples 1 to 3, the average tension (slidability in a drying process after wetting) was evaluated according to the following method.

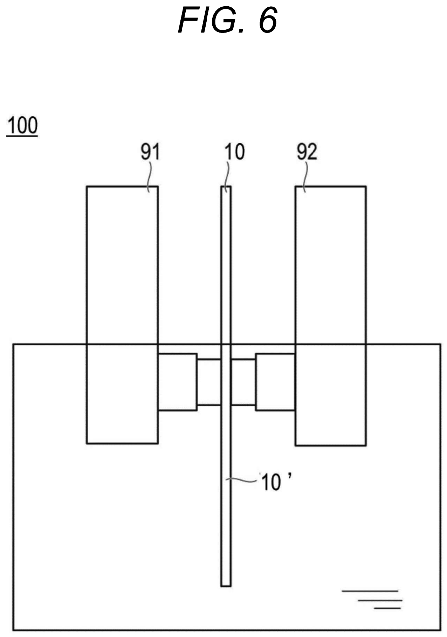

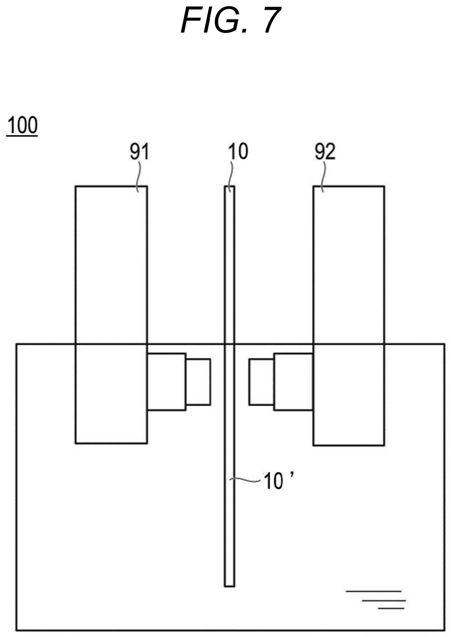

With reference to Figs. 6 and 7, a test device 100 for evaluating the sliding resistance (sliding test) of the medical device 10 according to the present embodiment will be described. Fig. 6 is a schematic view illustrating a test device 100 for performing a sliding test of the medical device 10 according to the present embodiment, and is a view illustrating a state in which the medical device 10 is sandwiched between a pair of contact members 91 and 92. Fig. 7 is a schematic view illustrating the test device 100 for performing a sliding test of the medical device 10 according to the present embodiment, and is a view illustrating a state in which the medical device 10 is separated from the pair of contact members 91 and 92.

As illustrated in Figs. 6 and 7, the test device 100 includes a pair of contact members 91 and 92. The pair of contact members 91 and 92 are configured to be able to approach and separate from each other, and can sandwich the medical device 10 with a predetermined force in a state in which the pair of contact members 91 and 92 approach each other. As the test device 100, DL1000 manufactured by OAKRIVER TECHNOLOGY Inc. may be used.

Next, a method of measuring the sliding resistance (average tension) of the sample (rod) 10' as the medical device 10 using the test device 100 will be described.

First, each sample (rod) 10' was immersed in tap water for 5 minutes, and then the sample 10' was taken out from the water, set in a pinch tester (DL1000 manufactured by OAKRIVER TECHNOLOGY Inc.) as the test device 100, and the pair of contact members 91 and 92 were brought close to each other to sandwich the sample 10' with a grip force of 500 gf. At this time, as the grip pads as the contact members 91 and 92, those made of silicone of Shore A60 and having a pad thickness of 12.35 mm were used.

Next, the resistance value was measured while pulling up the sample 10' at a predetermined speed. At this time, the speed at which the sample 10' was pulled up (test speed) was 8.3 mm/s, and the test stroke (pulling-up distance) was 25 mm.

Thereafter, the pair of contact members 91 and 92 were separated from each other to release the state in which the sample 10' was sandwiched, and then the sample 10' was returned to the initial position.

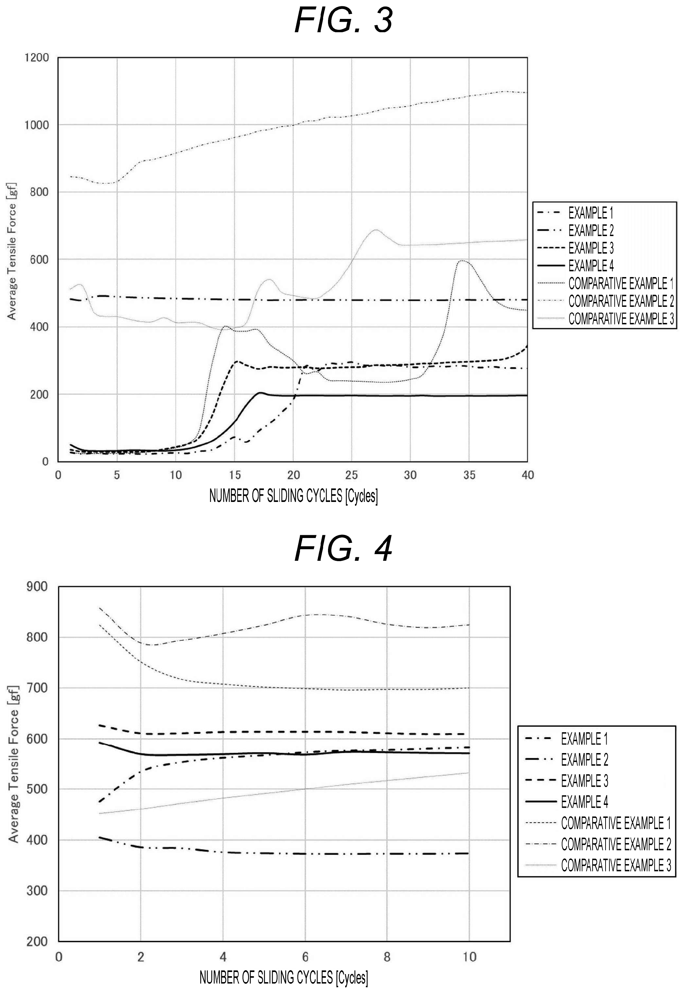

The above process was repeated a predetermined number of times, and the average tension (sliding resistance value) was measured a predetermined number of times. At this time, the number of times of measurement (number of sliding cycles) was set to 40, and the average tension was measured. As a result, the result as illustrated in Fig. 3 was obtained.

In the sample of Comparative Example 1 as illustrated in Fig. 3, a large peak was observed when the number of sliding cycles was around 15 and around 35, and in particular, when the number of sliding cycles was around 35, the value of the average tension was a very high value of about 600 gf. In addition, in the sample of Comparative Example 2, the value of the average tension showed a very great value exceeding 800 gf from the first sliding cycle, and further, in the sample of Comparative Example 3, a large peak was observed when the number of sliding cycles was around 18 and around 27, and the value of the average tension exceeded about 600 gf even after 27 cycles.

It is presumed that stickiness during use in a medical device having a lubricating coating is caused by such a rapid increase in average tension (the portion illustrating a clear peak) and a very large average tension, particularly a rapid increase in average tension. On the other hand, in the samples of Examples 1 to 4, such a clear peak was not observed, and the average tension value was also less than 500 gf over all the samples. That is, in the samples of Examples 1 to 4, it can be said that stickiness at the time of shifting from a wet state to a dry state is effectively suppressed.

### [Evaluation of sliding resistance in dry state]

For the samples (1) to (4) prepared in Examples 1 to 4 and the comparative samples (1) to (3) prepared in Comparative Examples 1 to 3, the average tension (slidability in a dry state) was evaluated according to the following method. In this evaluation, the same operation as in the above [Evaluation of sliding resistance in drying process after wetting] was performed except that the sample before the test was not brought into contact with water and the number of sliding cycles was changed.

Specifically, each sample was set in a pinch tester (DL1000 manufactured by OAKRIVER TECHNOLOGY Inc.) without being brought into contact with water, and sliding was performed 10 times at a grip force of 500 gf, a test speed of 8.3 mm/s, and a test stroke of 25 mm (as the grip pad, one similar to the above [Evaluation of sliding resistance in drying process after wetting] was used). The slidability was evaluated by measuring the average tension at the time of 10 sliding cycles. The results are illustrated in Fig. 4 and Table 2.

### [Evaluation of sliding resistance in wet state]

For the samples (1) to (4) prepared in Examples 1 to 4 and the comparative samples (1) to (3) prepared in Comparative Examples 1 to 3, the average tension (slidability in a wet state) was evaluated according to the following method. In this evaluation, the same operation as in the above [Evaluation of sliding resistance in drying process after wetting] was performed except that the test was performed while the sample was immersed in water.

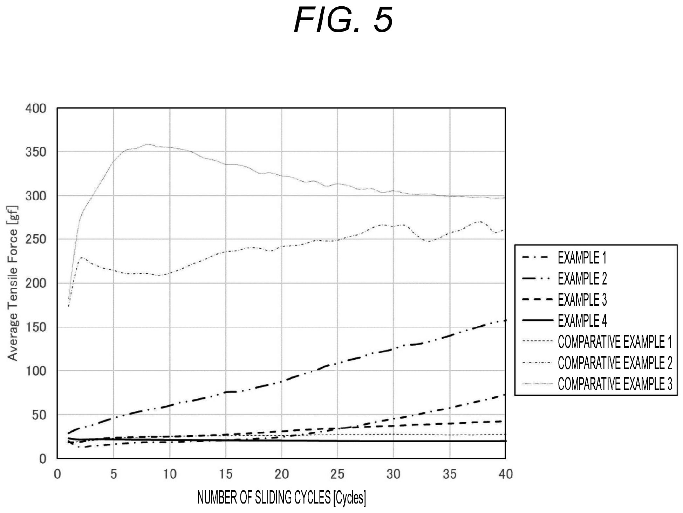

Specifically, each sample was set in a pinch tester (DL1000 manufactured by OAKRIVER TECHNOLOGY Inc.) in a state of being immersed in tap water, and sliding was performed 40 times at a grip force of 500 gf, a test speed of 8.3 mm/s, and a test stroke of 25 mm (as the grip pad, one similar to that in the above [Evaluation of sliding resistance in drying process after wetting] was used). The slidability was evaluated by measuring the average tension at the time of 40 sliding cycles. The results are illustrated in Fig. 5 and Table 2.

In both the dry state and the wet state, it is determined that the lower the average tension, the better the slidability.

**[Table 2]**

| | Silicone compound | | Block copolymer | Average tension [gf] | |
|---|---|---|---|---|---|
| | Type | Concentration in coating liquid [% by mass] | | Wet state | Dry state |
| Example 1 | Amino group-containing silicone (1) | 2.0 | p(GMA-b-DMAA) | 73.0 | 582.3 |
| Example 2 | Amino group-containing silicone (1) | 4.0 | p(GMA-b-DMAA) | 157.9 | 373.7 |
| Example 3 | Silicone compound (2) (mixture) | 0.57 | p(GMA-b-DMAA) | 43.0 | 609.9 |
| Example 4 | Silicone compound (2) (mixture) | 1.1 | p(GMA-b-DMAA) | 20.1 | 570.9 |
| Comparative Example 1 | - | - | p(GMA-b-DMAA) | 27.2 | **700.4** |
| Comparative Example 2 | Amino group-containing silicone (1) | 4.0 | - | **261.4** | 824.4 |
| Comparative Example 3 | Silicone compound (2) (mixture) | 4.0 | - | **297.9** | 531.2 |

In general, in a wet state, the hydrophilic block copolymer is more slippery than the silicone compound. That is, while the hydrophilic block copolymer exhibits good slidability, the slidability of the silicone compound decreases. Therefore, it is appropriate to compare Comparative Examples 2 and 3 using only a silicone compound in a wet state with Examples. Specifically, as illustrated in Fig. 5, in a case where the cover layer (surface lubricating layer) is formed using only a silicone compound (Comparative Examples 2 and 3), the maximum average tension measured in a wet state exceeds 200 gf. On the other hand, in a case where a cover layer (surface lubricating layer) is formed by using reactive silicone and a hydrophilic block copolymer (Examples 1 to 4), the average tension is 200 gf or less between the first sliding cycle and the 40th sliding cycle, and thus favorable slidability is exhibited. Further, in a case where the cover layer (surface lubricating layer) is formed by further using a non-reactive silicone in addition to the reactive silicone and the hydrophilic block copolymer(by using the reactive silicone, the non-reactive silicone, and the hydrophilic block copolymer) (Examples 3 and 4), the cover layer exhibits an average tension substantially equal to or less than that of the cover layer formed by using only the hydrophilic block copolymer (Comparative Example 1) (that is, more excellent slidability is exhibited).

On the contrary, in a dry state, there is a silicone compound that exhibits a very low sliding resistance value (average tension), and in general, the silicone compound tends to be more slippery than the hydrophilic block copolymer. Therefore, in a dry state, it is appropriate to compare Comparative Example 1 using only the hydrophilic block copolymer with Example. Specifically, as illustrated in Fig. 4, when the cover layer (surface lubricating layer) is formed using only the hydrophilic block copolymer (Comparative Example 1), the maximum average tension measured in a dry state is about 700 gf. On the other hand, in a case where a cover layer (surface lubricating layer) was formed by using reactive silicone and a hydrophilic block copolymer (Examples 1 to 4), the average tension was about 600 gf or less than 600 gf, and good slidability was exhibited.

To summarize the above, in the wet state (Fig. 5), the cover layer (surface lubricating layer) formed by using only a silicone compound (Comparative Examples 2 and 3) had extremely high average tension and poor slidability. From such results, it was expected that the cover layer (surface lubricating layer) contained a silicone compound, and thus the slidability deteriorated. However, according to Table 2 and Fig. 5, even when the cover layer (surface lubricating layer) contained a silicone compound, the samples of Examples 1 to 4 exhibited excellent slidability equivalent to that of the cover layer (surface lubricating layer) (Comparative Example 1) formed by using only the hydrophilic block copolymer.

In addition, according to Table 2 and Fig. 4 described above, in the dry state, the samples of Examples 1 to 4 were superior in slidability to the sample of Comparative Example 1 in which the cover layer (surface lubricating layer) was formed using only the hydrophilic block copolymer. Such an effect is considered to be caused by the silicone compound contained in the surface lubricating layer.

According to the present invention, a high lubricating property can be maintained without exhibiting adhesiveness even in a drying process after a medical device surface (surface lubricating layer) is in a wet state. Therefore, for example, when catheter treatment is performed by using a catheter or a guide wire, it is not necessary to keep wetting the surface every time operation is performed, or the number of times of spraying physiological saline or the like can be reduced. That is, according to the present invention, it is not necessary to perform an operation that becomes a work load during such a procedure, or the number of such operations can be reduced, so that it is possible to provide a medical device that reduces the work load during the procedure.

The present application is based on Japanese Patent Application No. 2023-205991 filed on December 6, 2023, the disclosure content of which is incorporated herein by reference in its entirety.

### Reference Signs List

- 10: Medical device
- 1: Base layer
- 1a: Base layer core portion
- 1b: Base material surface layer
- 2: Lubricating layer
- 100: Test device
- 10': Sample (rod)
- 91: Contact member
- 92: Contact member

## Claims

1. A medical device comprising: a base layer; and a surface lubricating layer supported on at least a part of the base layer, wherein
the surface lubricating layer contains a block copolymer having a structural unit (A) derived from a reactive monomer having an epoxy group and a structural unit (B) derived from a hydrophilic monomer, and a silicone compound, and
when 1.0 µL of purified water is added dropwise to the surface lubricating layer, and a water contact angle after 5 seconds from contact between a water droplet of the purified water and the surface lubricating layer is θ1 and a water contact angle after 5 minutes from the contact is θ2,
θ1 is 104° or more, and θ2 is 102° or less.

2. The medical device according to claim 1, wherein when a water contact angle after 1 minute from the contact between the water droplet of the purified water and the surface lubricating layer is θ3,
θ3 exceeds 102°.

3. The medical device according to claim 1 or 2, wherein the surface lubricating layer contains the silicone compound and the block copolymer at a mass ratio of 0.1 to 1 : 1.

4. The medical device according to claim 1 or 2, wherein the silicone compound contains reactive silicone.

5. The medical device according to claim 4, wherein the reactive silicone has at least one selected from the group consisting of a hydroxyl group (-OH), a carboxyl group, an amino group (-NH₂), an epoxy group, an ethylenically unsaturated group, and an alkoxysilyl group.

6. The medical device according to claim 1 or 2, wherein the reactive monomer having an epoxy group contains at least one selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether.

7. The medical device according to claim 1 or 2, wherein the hydrophilic monomer contains at least one selected from the group consisting of N,N-dimethylacrylamide, acrylamide, 2-hydroxyethyl methacrylate, and N-vinylpyrrolidone.

8. A method of manufacturing a medical device including a base layer and a surface lubricating layer supported on at least a part of the base layer, the method comprising:
preparing a coating liquid containing a block copolymer having a structural unit (A) derived from a reactive monomer having an epoxy group and a structural unit (B) derived from a hydrophilic monomer, a silicone compound, and a solvent; and
applying the coating liquid onto the base layer.

9. The method according to claim 8, wherein the coating liquid contains the silicone compound in an amount of 0.5 to 5% by mass.

10. The method of claim 8 or 9, wherein the silicone compound contains reactive silicone.

11. The method according to claim 10, wherein the reactive silicone has at least one selected from the group consisting of a hydroxyl group (-OH), a carboxyl group, an amino group (-NH₂), an epoxy group, an ethylenically unsaturated group, and an alkoxysilyl group.

12. The method according to claim 8 or 9, wherein the solvent contains at least one selected from the group consisting of methanol, ethanol, isopropyl alcohol, butanol, ethylene glycol, acetone, methyl ethyl ketone, cyclohexanone, ethyl acetate, butyl acetate, tetrahydrofuran (THF), butyl ether, dioxane, hexane, heptane, benzene, toluene, dimethyl sulfoxide (DMSO), and N,N-dimethylformamide (DMF).

13. The method according to claim 8 or 9, wherein the reactive monomer having an epoxy group contains at least one selected from the group consisting of glycidyl acrylate, glycidyl methacrylate, 3,4-epoxycyclohexyl methyl acrylate, 3,4-epoxycyclohexyl methyl methacrylate, β-methyl glycidyl methacrylate, and allyl glycidyl ether.

14. The method according to claim 8 or 9, wherein the hydrophilic monomer contains at least one selected from the group consisting of N,N-dimethylacrylamide, acrylamide, 2-hydroxyethyl methacrylate, and N-vinylpyrrolidone.
